(19) Europäisches Patentamt
European Patent Office
Office européen des brevets

(11) EP 0 926 241 A2

(12) EUROPEAN PATENT APPLICATION

(43) Date of publication:
**30.06.1999 Bulletin 1999/26**

(51) Int. Cl.[6]: **C12N 15/54**, C12N 15/82

(21) Application number: **98309592.8**

(22) Date of filing: **24.11.1998**

(84) Designated Contracting States:
**AT BE CH CY DE DK ES FI FR GB GR IE IT LI LU MC NL PT SE**
Designated Extension States:
**AL LT LV MK RO SI**

(30) Priority: **25.11.1997 GB 9724891**

(71) Applicant: **UNIVERSITY OF YORK**
**Heslington, York Y01 5DD (GB)**

(72) Inventors:
- **Bowles, Dianna Joy,**
  **The University of York**
  **Heslington, York Y01 5DD (GB)**
- **Calvert, Caroline Mary,**
  **The University of York**
  **Heslington, York Y01 5DD (GB)**
- **O'Donnell, Philip James,**
  **The University of York**
  **Heslington, York Y01 5DD (GB)**
- **Roberts, Michael Richard,**
  **The University of York**
  **Heslington, York Y01 5DD (GB)**

(74) Representative:
**Chapman, Paul William et al**
**Kilburn & Strode,**
**20 Red Lion Street**
**London WC1R 4PJ (GB)**

(54) **Use of dehydrodiconferyl alcohol glucosyl transferase in the regulation of plant development and defence**

(57) This invention relates to methods for inducing plant defence and resistance responses as well as regulating plant developmental events in monocots and dicots by modifying a gene encoding for a dehydrodiconiferyl alcohol glucosyl transferase (TWI1) isolated from wounded tomatoes.

EP 0 926 241 A2

## Description

This invention relates to methods for inducing plant defence and resistance responses and regulating plant developmental events. More particularly, this invention relates to methods for inducing the production of plant defence proteins such as pathogenesis-related (PR) proteins and proteinase inhibitor (pin) proteins and to methods for regulating resistance and acquired resistance to predators, insects, bacteria, fungi and viruses in plants, through manipulating levels of the plant hormones: salicylic acid, jasmonic acid, dehydrodiconiferyl alcohol glucoside, cytokinins and ethylene, and to methods for regulating developmental events that depend on these hormones, particularly, plant growth, reproduction and senescence.

Adaptation of a plant to its environment is brought about by recognition and response to external stimuli which cause changes in cellular activity. A chain of events link the initial recognition of the stimulus to changes in cells of the plant that ultimately lead to adaptation. These events constitute a signal transduction pathway, in which sequential molecular interactions transduce (lead) the signal from its perception through to the end-effects caused.

Plants respond to a vast range of environmental stimuli that include, for example: changes in their growing conditions (light, heat, cold, drought, water-logging etc); mechanical damage leading to injury, and challenge by pests and pathogens (herbivores, insects, fungi, bacteria, viruses etc). These stimuli lead to cellular events at the site(s) of perception, but also can trigger long-range events throughout the plant, leading to systemic changes. Thus, for example, in response to wounding and to pest/pathogen challenge, there are local and systemic events induced with signal transduction pathways occurring at the local site, systemic signal(s) communicating the local events around the plant, and signal transduction pathways occurring in distant cells that are responding to the systemic signal(s). Networking and cross-talk between intra- and inter-cellular signal transduction pathways is recognised to be an important means through which the plant integrates all of the information received from the environment.

Plant hormones play a central role in these induced responses to environmental stimuli, since they act as the intermediate molecular signals which trigger the transduction pathways leading from the external change(s) in the environment to the internal end-effect(s) within the plant. For example, in a variety of plant species, jasmonic acid is known to accumulate transiently during the wound response and has been implicated in transduction events linking mechanical injury to activation of wound-responsive genes. Another example is during the interactions of plants with pests and pathogens, when salicylic acid is known to increase in quantity (together with its precursor, benzoic acid and its volatile form, methyl salicylate) and is considered to be a central regulator of local and systemic acquired resistance and the activation of defence-related genes associated with resistance.

Whilst salicylic acid is a positive regulator of these induced resistance responses, there is evidence to show that the hormone is also a negative regulator of the wound response, such that if a plant is pre-treated with aspirin or salicylic acid, wound-responsive genes dependent on jasmonic acid for their expression are not induced [1,2,3,4]. This suggests there is communication between the signalling pathways induced by mechanical injury and induced by pests/pathogens, such that they do not occur simultaneously.

Senescence is the natural process which normally leads to cell death, either in a selected population of cells such as abscission cells or in whole organs such as flowers, leaves and fruits. This process of senescence can be developmentally regulated, such as, for example, in the ripening process, wilting and fading of flowers, yellowing and abscission of leaves. Alternatively, senescence and cell death can be induced by trauma, such as caused by, for example, chemicals, temperature extreme, pest and pathogen damage, disease or mechanical wounding.

The level of an active plant hormone in plant cells and tissues at any one time is dependent on the relative rates of its synthesis and degradation, the rates of transport to or from the cells/tissue, and the relative rates of its conversion to and from inactive metabolite(s). For plant hormones, this conversion to an inactive metabolite can involve the conjugation of the free active form of the hormone to a polar molecular species, such as a sugar, amino acid or peptide. Endogenous hormones made by the plant, and exogenous hormones applied to and taken up by the plant are subject to conjugation in this manner. When large quantities of exogenous hormones are applied to the plant the conjugation process has been likened to detoxification since it effectively clears the active hormones from the system rapidly.

Since the process of conjugation is known to be reversible in plants, at least for some hormones, it provides a flexible mechanism for regulating the pool size of active hormone in the absence of synthesis and degradation. Also, since most plant hormones are either apolar or amphiphilic, their reversible conjugation to a polar molecule provides a useful mechanism for containing the hormone on one side or other of a membrane, such as in the apoplast, or in a particular compartment of the symplast.

Whilst many different conjugates of plant hormones have been identified, a commonly found conjugate is the glucoside, formed through the transfer of glucose from a sugar nucleotide donor to the hormone via a β, O-glycosidic linkage. The enzymes responsible are O-glucosyl transferases and the available evidence indicates that each transferase is highly specific for the particular hormone it conjugates. For example, the glucosyl transferase responsible for conjugation of the plant hormone, indole 3-acetic acid, has been identified and shown to be specific for the auxin substrate [5]. Similarly, the glucosylation of salicylic acid has also been investigated, and an enzyme activity identified in a variety of plant species has been shown to be specific for salicylic acid and the sugar nucleotide donor, UDP-glucose [6].

Another important hormone is ethylene which is a gas under physiological conditions that influences a wide range of events in plants, including the regulation of growth, cellular differentiation and developmental processes. In particular, ethylene is the key regulator of senescence, which as stated above, is a genetically controlled process of degeneration normally leading to cell death and which occurs in specific cell-types and in whole organs such as flowers, leaves and fruits. The effects of ethylene on developmental processes are of considerable commercial importance. The effects can occur at low concentrations, whether the gas is produced by the plant itself or applied exogenously to the plant. Ethylene is also involved in defence/stress and resistance responses, such as directing how the plant combats challenges from pests and pathogens, and during the consequences of abiotic stimuli, for example, mechanical injury and water-logging. In these defence/stress and resistance responses, ethylene has a direct effect on the activation of specific genes, as well as a role in inducing cell death associated with hypersensitive responses.

Generally, ethylene is maintained at very low levels in plant tissue, but production can be rapid and massive during the senescence process, or during stress/trauma caused by biotic and abiotic stimuli. During the degenerative process of senescence, ethylene synthesis is regulated by positive feedback, such that one action of the ethylene produced is the up-regulation of the synthetic machinery and thus further production of more ethylene leading to an autocatalytic avalanche of increased levels of the hormone. In contrast, during stress/trauma, the "wound" or "stress" ethylene produced is regulated by negative feedback, leading to a hormone transient. Ethylene has been shown unequivocally to be a requirement for the developmental senescence process. The role of "wound" ethylene is less defined.

The ethylene signal transduction pathway is the most characterised of all plant hormones to date, with the identification of genes encoding a receptor, a negative regulator protein and a number of proteins implicated genetically in downstream events [7]. In contrast, very little is known of the regulation or the mechanism(s) by which ethylene levels rise in response to the environmental stimuli and to pest and pathogen attack.

Since plants have evolved inducible mechamisms of defence that respond to attack by pests and pathogens, there is considerable commercial interest in identifying methods of induction which will protect and even enhance the natural resistance of the crop plant. This is particularly relevant when the only agrochemicals available are hazardous both to the environment and the consumer. Often, during the natural course of a defence response to pest and pathogen challenge, a broad spectrum of defence-related genes and physiological events are induced in parallel and their success at conferring resistance arises from the multiplicity of their actions. Long-term efficacy of this strategy is much greater than that achieved by genetically modifying crop plants with single defence-related genes. This is because in field situations the alteration or insertion of a single defence-related gene can be overcome by pests and pathogens rapidly evolving and adapting to the single gene change. In addition, decreased crop yields and decreased product quality are features commonly encountered in resistant cultivars. Thus, there exists a strong requirement for new materials and processes to improve the resistance of plants under attack by pests and pathogens. This would preferably be through the induction of the plant's own defence systems.

There is also considerable commercial interest in identifying the molecular "switches" which respond to non-hazardous chemicals applied to the plant, and in turn, regulate developmental and defence responses, where and when applied. Whilst some inducible promoters have been found that are responsive to various chemicals, (e.g. PR gene promoters responsive to dichloroisonicotinic acid (DCINA) [8] which can be used to drive the expression of genes of interest), the range of applications envisaged could be increased dramatically if more promoters and more chemical inducers were identified. By way of background, DCINA is a widely used agrochemical which induces systemic acquired resistance (SAR) and is thought to act at a point downstream of salicylic acid in the transduction pathway leading to SAR gene expression.

For plant resistance and post-harvest protection of raw material quality, the speed of the defence response mounted by the plant cells/tissues, often determines the overall success-rate. Therefore, major interest lies in identifying rapidly responding promoters and, dependent on the application, those that are either capable of driving expression in a wide range of cell-types or those that are highly specific to a particular cell-type or tissue, for example, epidermal cells or leaves, but not stems, etc.

There is also major commercial interest in identifying ways in which senescence can either be controlled, prevented completely, the time-span of senescence regulated, or its occurrence induced only when required. Since senescence is intimately associated with ethylene, the problems of senescence are really problems of ethylene quantity and ethylene action. For example, in the post-harvest care of fruits, vegetables and flowers, cuts and bruising can stimulate ethylene production which in turn causes cell death in the traumatised tissues as well as affecting the adjacent fresh produce. This in turn leads to massive losses in the quality of these materials during transportation and storage. Traditional technologies addressing post-harvest issues have been tried for decades but suffer from problems of side-effects, toxicity, high costs and an inability to shut down completely ethylene synthesis **[9]**.

In the present invention, a gene TWI1, is now known to code for a glucosyl transferase, specifically dehydrodiconiferyl alcohol (DCA) glucosyl transferase (GTase) which regulates levels of a key signalling intermediate, dehydrodiconiferyl alcohol glucoside (DCG). The function of TWI1 gene as a DCA GTase was never previously disclosed. Through detailed analyses of the expression patterns of TWI1 in tomato, this gene is believed to function in those signalling pathways leading to developmental and defence responses controlled by ethylene. Using transgenic plants with modified levels of TWI1 expression, we demonstrate a key role for this gene in plant responses to wounding and to pathogens.

A disclosure of a partial sequence of the TWI1 cDNA on an EMBL database of a wounded tomato leaf library did not indicate that TWI1 encoded DCA GTase nor did the EMBL database indicate the role of this gene in the signalling pathways of plants. These factors can not be deduced from the partial sequence disclosed, nor from the source of the mRNA from which the TWI1 cDNA was derived. This invention, therefore, provides the first-ever correlation between the action of DCA GTase and the regulation of DCG and ultimately of ethylene, a common intermediate in many diverse processes in plants.

Accordingly, the present invention provides a method of altering the signalling pathways of a plant involving altering the levels of dehydrodiconiferyl alcohol glucoside, ethylene, jasmonic acid and/or salicylic acid present in the plant during plant defence and development. The method comprises regulating and/or interfering with, altering, or inhibiting the normal functioning of the TWI1 gene encoding DCA GTase in plants. This is particularly useful in tomato plants, however, this invention would apply with similar advantage to other dicotyledonous or monocotyledonous plants (i.e. broad-leaved plant species as well as grasses and cereals). This invention is applicable to any horticultural or agricultural species, including those in which fruit-ripening and/or post-harvest storage are important considerations.

According to one aspect of this invention, there is provided a recombinant or isolated DNA molecule which encodes for a DCA glucosyl transferase in plants. In preferred embodiments, the DCA GTase gene (TWI1) comprises the nucleic acid sequence or at least portions (or fragments) of the nucleic acid sequence shown in FIG 1 or the amino acid sequence of FIG 3. Also nucleic acid molecules that hybridise to one or more of nucleic acid molecules of the TWI1 gene of FIG 1 are also within the scope of this invention. Such nucleic acid molecules are referred to herein as "hybridising" nucleic acid molecules. Desirably hybridising molecules of the present invention are at least 10 nucleotides in length and preferably are at least 25, at least 50, at least 100, or at least 200 nucleotides in length.

A hybridising nucleic acid molecule of the present invention may have a high degree of sequence identity along its length with a nucleic acid molecule complementary to the TWI1 gene (e.g. at least 50%, at least 75%, at least 90%, at least 95% or at least 98% identity), although this is not essential. The greater the degree of sequence identity that a given single stranded nucleic acid molecule has with another single stranded nucleic acid molecule, the greater the likelihood that it will hybridise to a single stranded nucleic acid molecule that is complementary to that other single stranded nucleic acid molecule under appropriate conditions.

Preferred hybridising molecules hybridise under conditions of moderate or high stringency. Hybridisation conditions are discussed in detail at pp 1.101 - 1.110 and 11.45 - 11.61 of Sambrook *et al* [*Molecular Cloning*, 2nd Edition, Cold Spring Harbor Laboratory Press (1989)]. One example of hybridisation conditions that can be used involves using a pre-washing solution of 5 x SSC, 0.5%SDS, 1.0mM EDTA (pH 8.0) and attempting hybridisation overnight at 55°C using 5 X SSC. However there are many other possibilities. Some of these are listed in Table 1 of WO98/45435, for example. (See especially the conditions set out under A-F of that table and, less preferably those listed under G to L or M to R.)

Another approach is to determine the Tm for a given perfect duplex (i.e. with no mismatches) of a certain length under given conditions and then to perform attempted hybridisation with a single strand of the duplex under those conditions, but at a temperature sufficiently below the Tm to allow for formation of a range of stable hybrids at an acceptable rate, whilst still requiring a reasonable degree of hybridisation specificity. The Tm for such a duplex can be determined empirically by providing the duplex and gradually increasing the temperature until the Tm is achieved. The Tm can also be estimated e.g. by using: $Tm = 81.5 + 16.6 (\log_{10} [Na+]) + 0.41(\text{fraction } G + C) - (600/N)$, where N is the chain length. (This formula is reasonably accurate for Na+ concentrations of 1M or less and for polynucleotide lengths of 14 to 70, but is less accurate when these parameters are not satisfied.) For nucleic acid molecules of greater than 200 nucleotides in length hybridisation may, for example, be carried out at 15 to 25°C below the Tm of a perfect hybrid (i.e. with no mismatches) under given conditions. However as the length is decreased the Tm is lowered so that it is sometimes inconvenient to carry out hybridisation at Tm - 25°C. Hybridisation with shorter nucleic acid molecules is therefore often carried out at only 5 to 10°C below the Tm. Moderate or high stringency conditions will usually only allow a small proportion of mismatches. As a rule of thumb, for every 1 % of mismatches there is a reduction of Tm by 1- 1.5°C. Preferably hybridisation conditions are chosen to allow less than 25 % mismatches, more preferably to allow less than 10% or less than 5% mismatches. Hybridisation can be followed by washes of increasing stringency. Thus initial washes may be under conditions of low stringency, but these can be followed with higher stringency washes, up to the stringency of the conditions under which hybridisation was performed.

The foregoing discussion of hybridisation conditions is provided for general guidance but is not intended to be limiting. This is because a skilled person will be able to vary parameters as appropriate in order to provide suitable hybridisation conditions, and can take into account such variables as polynucleotide length, base composition, nature of duplex (i.e. DNA/DNA, RNA/RNA or DNA/RNA), type of ion present, etc.

The term "portions" or "fragments" as used herein should be interpreted to mean that a sufficient number of nucleic acid or amino acid residues are present for the fragment to be useful (i.e. to act as or encode a DCA GTase, or to be of a size useful as a probe). Typically, at least four, five, six, ten up to 20, 100 or even up to 500 or more residues may be present in a fragment. Useful fragments include those which are the same as or similar to or equivalent to those naturally produced by the TWI1 gene or its equivalent gene and enzyme in other plants, for example, as in FIGS 4 and 5 for tobacco and rice, respectively.

As used in the present application, similarity between nucleic acid and/or amino acid molecules may be defined as having "homology" or as being a "homologue" to a reference molecule. In this case the reference molecule is the TWI1 gene of FIGS 1 and 2. Substantial sequence homology is defined herein as having a close structural relationship between nucleotides or amino acids. For example, substantially homologous DNA sequences may be 60% homologous, preferably 80% and most preferably around 90 to 95% homologous, or more, and substantially homologous amino acid sequences may preferably be 35%, more preferably 50%, most preferably more than 50% homologous. Homology also includes a relationship wherein one or several subsequences of nucleotides or amino acids are missing, or subsequences with additional nucleotides or amino acids are interdispersed. When high degrees of sequence identity are present there may be relatively few differences in the amino acid sequences. Thus, for example, they may be less than 20, less than 10, or even less than 5 differences in amino acid sequences.

The degree of nucleic acid and/or amino acid sequence identity can be calculated, for example, using a program such as "BESTFIT" (Smith and Waterman, *Advances in Applied Mathematics*, pp. 482-489 (1981)) to find the best segment of similarity between any two sequences. The alignment is based on maximising the score achieved using a matrix of amino acid similarities, such as that described by Schwarz and Dayhof (*Atlas of protein Sequence and Structure*, Dayhoff, M.O., pp. 353-358). Additionally, sequence identity may be determined, for example, by comparing sequence information using the GAP computer program, version 6.0 described by Devereux *et al* (*Nucl. Acids Res.* **12** 387 (1984)) and available from the University of Wisconsin Genetics Computer Group (UWGCG). The GAP program utilises the alignment method of Needleman and Wunsch (*J. Mol. Biol.* **48** 443 (1970)), as revised by Smith and Waterman (*Adv. Appl. Math* **2** 482 (1981)). The preferred default parameters for the GAP program include: (1) a comparison matrix of Gribskov and Burgess, *Nucl. Acids Res.* **14** 6745 (1986), as described by Schwartz and Dayhoff, eds., *Atlas of Protein Sequence and Structure,* National Biomedical Research Foundation, pp. 353-358 (1979); (2) a penalty of 3.0 for each gap and an additional 0.10 penalty for each symbol in each gap; and (3) no penalty for end gaps. Where high degrees of sequence identity are present there may be relatively few sequence differences. Thus for example there may be less than 20, less than 10, or even less than 5 differences.

Using the TWI1 cDNA sequence as a probe to analyze expression of the DCA GTase during a pathogen response in tomato, we observed that the gene is induced during gene-for-gene mediated resistance (R) response involving the Cf9 R gene to *Cladosporium fulvum*. Similarly, using a homologous DCA GTase gene which we isolated from tobacco as a probe (FIG 4), we also found induction during gene-for-gene mediated R response involving the N gene to tobacco mosaic virus (TMV). In this latter system, induction of the GTase gene in response to TMV was causally dependent upon the elevation of salicylic acid. These data imply a role for the TWI1 gene product in salicylic acid-mediated pathogen responses. Thus, an object of this invention is the use of TWI1 gene in stimulating or improving pathogen related responses in plants, through induction of the DCA GTase.

In transgenic tobacco plants which constitutively express the DCA GTase at a high level, we found that the formation of necrotic lesions and the induction of PR-gene expression in response to the bacterial elicitor, harpin **[10]**, is completely suppressed. In contrast, in plants in which DCA GTase expression is repressed, the response to harpin is enhanced. These data show that the DCA GTase gene product impacts directly on events at the local site of challenge with consequences on the process of acquired resistance.

Importantly, the implication is that in transgenic plants expressing an antisense gene to the DCA GTase, a hypersensitive response (HR) and acquired resistance to pathogen challenge may be enhanced. This forms a further object of this invention.

In the wound response, JA and ethylene are causally required for proteinase inhibitor gene expression. The elevation of endogenous JA is very rapid and transient and is dependent on ethylene action [**15**]. Salicylic acid applied to plants prior to wounding inhibits this elevation in JA completely and also inhibits pin gene expression. The wound induction of the DCA GTase is also very rapid with a parallel time-course to the elevation in JA. In plants expressing the TWI1 antisense gene, wounding does not induce pin2 expression. Expression of the gene encoding ethylene-forming enzyme occurs as normal, but in contrast to wild-type plants, in the transgenic plants the down regulation no longer occurs. This provides further direct evidence for the role of the DCA GTase gene product in the regulation of ethylene and ethylene-dependent responses.

DCA GTase activity as it relates to ethylene has never previously been identified nor contemplated. This would not be an obvious occurrence despite existing literature on GTases. Moreover, since the function of the gene was not previously available for relevant antisense experiments, the opportunity to analyze the effects of DCA GTase down-regulation on ethylene did not exist prior to this disclosure. In this invention, we utilise antisense technology to demonstrate that down-regulation of DCA GTase leads to prolonged levels of stress ethylene. Therefore, a further aspect of this invention is the use of the aforementioned TWI1 or any functional homologues thereto for use in down regulating DCA GTase in a plant of interest.

According to a further aspect of this invention, there is provided antisense nucleic acid which includes a transcribable strand of DNA complementary to at least part of the strand of DNA that is naturally transcribed in a gene encoding DCA GTase. This involves the construction of transformation vectors possessing either the entire or partial coding sequence of the homologous GTase gene from the species to be transformed in the reverse orientation, under the transcriptional control of a constitutive promoter such as the Cauliflower Mosaic Virus 35S promoter and a transcription terminator sequence such as the *Agrobacterium tumefaciens* nos terminator. Also present in the vector, it is preferable, but not necessary, to include a plant selectable marker gene which enables a plant transformed with the TWI1 gene or a gene substantially homologous thereto, to be distinguished from plants not so transformed. Such markers may include, for example, neomycin phosphotransferase II or hygromycin phosphotransferase. Typically these selective markers are for antibiotic or herbicide resistance. Vectors containing these sequences may either be broad host range binary vectors useful for *Agrobacterium*-mediated transformation such as those derived from pBin19 [13], or standard *E. coli* vectors useful for production of high levels of plasmid for transformation mediated by particle delivery. In addition to the tomato DCA GTase antisense construct described in the examples below, we have also produced an antisense construct using sequences from the tobacco DCA GTase gene of **FIG. 4**.

To achieve over-expression of the DCA GTase, the coding sequence or portion of the coding sequence of the tomato TWI1 cDNA, or a coding sequence encoding an active homologous DCA GTase isolated from any other organism or a nucleic acid sequence synthetically produced by means well-known to those skilled in the art, may be placed under the control of promoters activated specifically in the tissues of interest, these being preferably ripening fruit and senescing leaves or flowers, and followed by a transcription terminator sequence.

In the present invention, through use of Northern analyses, we show for the first time that a high expression of DCA GTase exists in senescence and in ripened fruits. The implications of this are great, namely there may be an increased "need" for the DCA GTase in ethylene-mediated events. It is therefore an object of this invention to regulate levels of expression of DCG and therefore regulate the processes of senescence and fruit ripening.

On an application point, it is easier to obtain the over-expression effects in any plant species since a heterologous gene will produce the same effects. For an antisense approach, the homologous gene is preferred and may be isolated for any commercially important plant or crop.

Another aspect of this invention is the use of a promoter comprising the 5' upstream region of the TWI1 DCA GTase gene. The promoter claimed under this invention or one similar (substantially homologous) to that of FIG 2 isolated from plants other than tomato would exhibit activation characteristics including rapid activation following mechanical wounding or pathogen attack, including activation by salicylic acid, various salicylic acid analogues thereof and the functionally-related compound, DCINA. We show that the wound induction of the TWI1 gene and the induction by the chemical elicitors is via two independent pathways. Also, promoters of homologous DCA GTase genes from other plant species exhibiting similar activation characteristics in their respective species are also claimed by way of this invention.

Activation of the DCA GTase at the appropriate times using promoters derived from other sources is also claimed within the scope of this invention ((e.g. at the onset of senescence; e.g. *Arabidopsis* SAG12 promoter **[11]**) or at the onset of ripening (e.g. tomato polygalacturonase promoter **[12]**)).

The promoters in the present invention were isolated from clones obtained from a commercial genomic library of tomato by using the TWI1 cDNA sequence as a probe. Sequencing of such clones identified the DCA GTase coding sequence, with the 5' upstream region to approximately 5 kilobases of the DCA GTase transcription start sequence considered to be the promoter. Promoters of similar or substantially homologous GTase genes to the TWI1 gene of wounded tomato plants may be isolated from other genomic plant libraries or by utilising isolation techniques well-known to those skilled in the art, including, for example, the use of inverse polymerase chain reaction.

The promoter from the TWI1 gene is useful as a sequence capable of regulating the rapid accumulation of desirable gene products at sites of physical injury to a plant. Gene products considered desirable for such control include, but are not limited to: polypeptides with anti-microbial, antifungal, anti-insect etc activities, or polypeptides which have an activity which would protect the plant from further damage, for example, by altering cell wall synthesis activities. The promoter would also be useful in driving the regulated expression of a particular gene product following application of SA or SA analogues to the plant. The TWI1 promoter of FIG 2 could similarly be used to drive the expression of other wound inducible genes substantially homologous to the TWI1 gene in plants other than tomatoes, such as in dicotyledonous and monocotyledonous plants.

It is yet another aspect of this invention to provide transformed host cells comprising recombinant DNA encoding a plant DCA GTase in operable linkage with expression signals including promoter and termination sequences which permit expression of said DNA in the host cell. Preferably, DNA is transformed into plant cells using a disarmed Ti-plasmid vector and carried by *Agrobacterium* in procedures known in the art, for example as described in EP-A-0116718 and EP-A0270822. Alternatively, the foreign DNA could be introduced directly into plant cells using electrical discharge apparatus. This method is preferred where *Agrobacterium* is ineffective, for example, where the recipient plant is monocotyledonous. Any other method that provides for the stable incorporation of the DNA within the nuclear DNA of any plant cell of any species would be suitable. This includes species of plants which are not currently capable of genetic transformation.

The present invention also includes the production of transgenic plants (or parts of them, such as propagating material) containing DNA in accordance with the invention as described above. The constructs would include a promoter and coding sequence from, for example, the tomato TWI1 gene, or another promoter and coding sequence of plant DCA GTase exhibiting an analogous activation pattern for the purpose of regulated expression of desirable gene products at sites of attack or following elicitor application. Further, transgenic plants containing the TWI1 coding sequence, other sequence encoding a homologous plant GTase, or fragments thereof, in sense or antisense orientation, under the control of constitutive, developmental or tissue-specific promoters for the purpose of altering the natural levels of DCA GTase activity are also within the scope of this invention.

Transgene constructs are produced preferably using available promoters and terminator sequences from standard *E. coli* cloning vectors in combination with a GTase coding sequence (such as FIGS. 1, 2, 4 or 5). Constructs can either then be cloned into other *E. coli* vectors containing plant selectable marker genes, either to be used directly for particle bombardment transformation, or for *Agrobacterium*-mediated transformation when a binary vector will be used.

Another aspect of this invention is activation of specific enzyme activity, namely DCA GTase. The activation of DCA GTase by transfer of sequences encoding DCA GTase to other species will not necessarily be species-dependent. For down-regulation strategies however, it would be a preferred method to use the homologous gene from the target species to enable an efficient antisense effect.

The invention also provides a method for improving the resistance of plants to a very broad spectrum of pests and pathogens by regulating the levels of key signalling intermediates and thus increasing the natural defence responses of plants to any challenge. In particular, this invention will benefit crops growing in the field and benefit post-harvest care and protection of plant products. For instance, increased basal levels of an unconjugated intermediate in DCA GTase antisense plants could induce a "resistant state". In ethylene-mediated senescence, this invention seeks to improve the shelf-life/vase-life of products, whether fruit, vegetables, cut flowers, leaves, pot plants etc. This invention also teaches a method of controlling the senescent process, i.e. inducing ripening at a particular time in response to a spray or changed condition.

The following non-limiting examples are provided as an illustration of the usefulness of the above-described invention, wherein reference is made to the following figures:

FIG. 1 The cDNA sequence for the DCA GTase encoded by the TWI1 gene, isolated from tomato.

FIG. 2 The 5' upstream region for the TWI1 gene up to the start codon and including the promoter region.

FIG. 3 Amino acid (DCA GTase) sequence of the TWI1 gene.

FIG. 4 Nucleic acid sequence for a homologous GTase enzyme isolated from tobacco.

FIG. 5 Nucleic acid sequence for a homologous GTase enzyme isolated from rice.

FIG. 6 Reference gel demonstrating TWI1 expression during stages of tomato fruit development. RNA extracted from tomato fruits at different developmental stages and subjected to Northern Blotting and probed with TWI1 cDNA are shown. Lane 1: immature green fruit; Lane 2: mature green fruit; Lane 3: breaker stage; Lane 4: pink-ripe; and Lane 5: red-ripe fruit.

FIG. 7 Reference gel showing the expression of the proteinase inhibitor (pin)2 gene and ethylene-forming enzyme (ACO) in transgenic tomato lines expressing a TWI1 antisense gene.

FIG. 8 Reference gel electrophoresis demonstrating the accumulation of TWI1 mRNA (DCA GTase) during wounding and elicitor treatment in tomato plants. Each lane of gel is described in full in Example 2.

FIG. 9 Reference gel demonstrating a time-course of TWI1 mRNA accumulation by wounding and salicylic acid (2 mM) treatment in tomato. Each lane is described in full in Example 3.

FIG. 10 Reference gel showing that wound-induced TWI1 expression is normal in nahG plants, and thus SA-independent.

FIG. 11 Reference gel demonstrating local and long range expression of TWI1 on wounding tomato plant leaves.

FIG. 12 Reference gels demonstrating TWI1 mRNA accumulation in cotyledons of tomato plants injected with either intercellular fluid containing the Avr9 avirulence protein from *Cladosporium fulvum* (IF9) or water. Tomato plants carrying the Cf9 resistance gene (Cf9) or without the resistance gene (Cf0) were used.

FIG. 13 Tobacco TWI1 expression in response to TMV infection in tobacco NN plants. The reference gels show the accumulation of the tobacco TWI1 homologue mRNA in TMV-infected wild-type tobacco plants, but no accumulation in mock (water) inoculated plants, nor in plants transgenic for nahG salicylate hydroxylase gene. The time course indicates time after transfer of plants from 30°C to 24°C at which point the resistance response is initiated.

FIG. 14 Histograms indicating the levels of the free and conjugated salicylate present in leaves of the same plants as for Fig. 13, at the time-points (hours) indicated on the x-axis. Significant SA accumulation only occurs in wild-type NN tobacco, but not NN+ NahG tobacco.

FIG. 15 Reference gel showing tobacco acidic chitinase (PR3A) accumulation in water- or harpin-treated tobacco leaves at either 1, 2 or 3 days after injections, and in healthy (H) leaves. The plants are either wild-type (WT) or transgenic for over-expression (OVER) or antisensed (ANTI) of the tobacco DCA GTase gene.

FIG. 16 HPLC traces showing elution profiles of compounds from glucosyl transferase assays conducted using recombinant GST fusion proteins, DCA and UDP-glucose. The fusion proteins tested were tobacco GTase and tomato annexin. A novel peak ("Peak A") is produced only by the tobacco GTase fusion protein, not in the GST-annexin control, and this peak represents dehydrodiconiferyl alcohol glucoside (DCG).

FIG. 17 HPLC traces showing elution profiles of compounds from glucosyl transferase assays conducted using recombinant GST fusion proteins, DCA and UDP-glucose. The fusion proteins tested were tomato TWI1 GTase and tomato annexin. Three peaks are produced specifically by the tomato GTase fusion protein, Peak A representing DCG. Peaks B and C represent other isoforms of conjugated DCA.

EXAMPLE 1

The TWI1 (tomato wound-inducible 1) gene was first identified and analyzed as a partial cDNA from a differential screen of a tomato-wounded-leaf cDNA library.

Using the partial cDNA as a probe in Northern analyses TWI1 mRNA was confirmed as wound-inducible, with transcripts detectable by 15 minutes after injury to the leaves. Expression of the gene corresponding to TWI1 was also found to be developmentally-regulated. Whilst not expressed in unwounded leaves of a young tomato plant, TWI1 mRNA became abundant in older yellow leaves and was also found at high levels in red-ripe tomato fruit **(FIG 6)**. The pattern of induction of TWI1 by elicitors of other wound - responsive genes was also analyzed. The TWI1 was observed to be induced by plant cell wall fragments and salicylic acid, suggesting at the time (P J O'Donnell, 1995, Doctoral Thesis from Leeds University) TWI1 belonged to the family of defence-related proteins, know as the pathogenesis-related (PR) proteins, all of which are induced by salicylic acid and some of which are also induced by mechanical injury.

When a full-length cDNA of TWI1 was obtained and sequenced, homology was found to a large family of previously identified sequences encoding glucosyl transferases. The closest homology to existing sequences was observed to be that of *M. esculenta* Crantz cDNAs, mecgt1, encoding a UDP-glucose glucosyl transferase (54.3%) and mecgt5 encoding a UTP-glucose glucosyl transferase (52.2%). These have been identified as transferases involved in glucosylation of secondary metabolites. High homology was also found to a glucosyl transferase of *Zea mays*, involved in the glucosylation of IAA (52.8%) and to a ripening-related glucosyl transferase of tomato (ERT1B) known to glucosylate secondary metabolites.

The standard approach to identifying function is to use an antisense strategy, in which a transgene is constructed which expresses the gene of interest in antisense orientation, thereby leading to constitutively negligible levels of the gene product. The phenotype of the plants can then be analyzed to determine the effects of knocking out the expression of the gene. Using this antisense technology, tomato plants were transformed with TWI1 coding sequence in antisense orientation whose expression was constitutively driven by a Cauliflower Mosaic Virus (35S) promoter. A 480bp fragment from the 5' end of the TWI1 cDNA clone was produced by Polymerase Chain Reaction using the following primers:

5' primer - TCTTTCCTCTAGAATGCAAGGTC
incorporating a *Xba*I restriction site
3' primer - GTTCAGGTACCGATGACACATTC
incorporating a *Kpn*I restriction site

When digested with *Xba*I and *kpn*I, a 461 bp fragment was produced. This was sub-cloned into *Xba*I/*Kpn*I digested site of the binary vector pJR1Ri, giving a construct with the TWI1 fragment in the antisense orientation. After transformation into *E. coli*, the plasmid was transferred into the *Agrobacterium* strain LBA4404, by triparental mating. Cultures were then selectively grown up of *Agrobacterium* containing the plasmid. This was then used to transform tomato plants via *Agrobacterium*. Selection of potential transformed plants was on the basis of resistance to kanamycin. Regenerated plants were studied by RNA analysis to investigate the effect of the TWI1 antisense transgene.

It was discovered in three independently transformed primary transformants that the response to injury had changed. Four hours after wounding, the standard wound-response gene marker, proteinase inhibitor was expressed at low levels, whereas the gene encoding ethylene-forming enzyme (ACO), normally expressed transiently was not down-regulated and wound ethylene levels were maintained at high levels **(FIG 7)**.

The revelation that TWI1 was wound-inducible in no way implicates the gene product in a regulatory role, quite the opposite. This gene is responding to wound-induced signals. The fact the TWI1 gene shared homology to other genes encoding known glucosyl transferases would not implicate the gene product in a regulatory role, since many glucosyl transferases merely glucosylate secondary metabolites such as ERT1B **[14]**.

EXAMPLE 2

Accumulation of TWI1 mRNA by mechanical wounding and elicitor treatments was assessed. Results can be seen in **FIG 8**. Wounding was carried out by crushing the terminal leaflets of 21 day old tomato plants (*Lycopersicon esculentum* Mil.) cultivar Moneymaker, with a pair of tweezers. For all elicitor treatments 21 day old plants were excised at the base of the stem and incubated for 30 minutes in the various treatments, at the stated concentrations. After 30 minutes in the elicitor, the plants were transferred into water for the remainder of the incubation period. For all treatments, plants were maintained under constant light at 22°C. Leaf material was harvested at 1 hour after wounding/treatment in each case, and total RNA was then extracted. 10 µg of total RNA from each sample was separated by agarose gel electrophoresis in gels containing 7% formaldehyde, blotted onto Hybond-N membrane and probed with $P^{32}$ labelled TWI1 cDNA. In FIG. 8 the results are demonstrated, with the labels being: Healthy, leaves from non-excised plants; Wound, leaves 1 h post-wounding; $H_2O$, plants excised and incubated in water only; JA, jasmonic acid (100 mM); Systemin, synthetic systemin peptide (100 nM); SA, salicylic acid (2 mM); ASA, acetyl salicylic acid (2 mM); BA, benzoic acid (2 mM); 3,4 di-OH BA, 3,4 dihydroxybenzoic acid (2 mM); 2,6 di-OH BA, 2,6 dihydroxybenzoic acid (2 mM); DCINA, 2,6 dichloroisonicotinic acid (1 mM). The filter was exposed to film for 3 days. Equal loading was confirmed by re-hybridising the stripped blot with a $^{32}P$ labelled ribosomal RNA probe.

EXAMPLE 3: Time Course of Expression of TWI1 and Response to Wounding And Salicylic Acid

**FIG. 9** is a Northern analysis comparing the time-course of induction of DCA GTase and PR1a (PR= pathogenesis-related) gene expression following application of SA to the tomato plants through the transpiration stream. The increase in steady-state levels of the DCA GTase transcripts is very rapid when compared to those of PR1a, and become detectable within 10-15 minutes of treatment. To our knowledge, this is the most rapid SA-responsive gene so far identified, since the kinetics of up-regulation of SAR genes in tobacco and tomato are all known to be comparable to that of the PR1a shown in **FIG. 9**.

The time-course of TWI1 mRNA accumulation by wounding and salicylic acid (2 mM) treatment is specifically demonstrated in **FIG. 9**, including the induction of PR1 by SA treatment. 21 day old tomato plants were wounded, or excised at the base of the stem and incubated with 2 mM salicylic acid, as described in Example 2. Leaf material was harvested at each time point shown, and total RNA extracted. 10 µg of total RNA was fractionated through an agarose gel containing 7% formaldehyde, blotted onto Hybond-N membrane and probed with $^{32}P$ labelled TWI1 cDNA, or PR1 cDNA. Time shown in **FIG. 9** is in hours after wound/SA application. The filters were exposed to film for 24 hours (TWI1 filters) or 7 days (PR1 filters).

To assess whether SA might be a wound-induced signal which induced TWI1 expression, we also wounded leaves of transgenic tomato plants harbouring the salicylate hydroxylase gene (NahG), which presents SA accumulation. As shown in **FIG 10**, the response of TWI1 to wounding in NahG tomato plants is not significantly different from the response in wild-type plants, indicating that wound induction of this gene is SA-independent.

EXAMPLE 4: Local and Systemic Wound Induction of TWI1 mRNA

**FIG. 11** compares the timing of wound-induction GTase expression in the leaf that is damaged and in the systemically responding undamaged leaf of the plant.

In **FIG. 11**, mechanical wounding was carried out on the terminal leaflets the first leaf of 21 day old tomato plants, as described in Example 2. Leaf material was harvested from wounded leaf (local) and the unwounded leaf 2 (systemic) at the stated times, and total RNA extracted. 10 µg of total RNA was separated through an agarose gel containing 7% formaldehyde, blotted onto Hybond-N membrane and probed with $^{32}P$ labelled TWI1 cDNA. Time given is hours after mechanical wound. The hybridised filter was exposed to film for 8 days.

EXAMPLE 5

The expression of TWI1 in response to gene-for-gene mediated pathogen resistance was assessed using Avr9-containing extracts to elicit a resistance response in tomato harbouring the Cf9 resistance gene. Elicitor was injected into cotyledons of Cf9 plants or control plants with no Cf9 gene (Cf0) and as a further control, Cf9 cotyledons were injected with water. At various time-points after injection, RNA was extracted and subjected to Northern blotting using the TWI1 cDNA as a probe (**FIG 12**). Significant induction of TWI1 expression was only detected in Cf9 plants injected with Avr9 elicitor, demonstrating a pathogen-resistance response-specific activation of the GTase.

EXAMPLE 6

The expression of the tobacco TWI1 homologue during a pathogen-resistance response was investigated in tobacco plants harbouring the N-resistance gene infected with tobacco mosaic virus (TMV). Wild-type tobacco, or tobacco transformed with a salicylate hydroxylase gene (NahG) which cannot accumulate salicylic acid (SA), were inoculated with TMV or mock-inoculated with water and grown for 2 days at 30°C to permit virus spread. The plants were then transferred to 24°C to initiate the resistance response and RNA, SA and SA conjugates were extracted at various time-points. As shown in **FIG 13**, GTase expression was only induced in wild-type NN tobacco infected with TMV. No GTase expression was observed in the NahG transformants which did not significantly accumulate SA, whereas expression in wild-type plants correlated with the timing of SA production (**FIG 14**).

EXAMPLE 7 - Production of transgenic tobacco plants

Plasmid constructs containing the tobacco TWI1 GTase homologue in either sense or antisense orientation were produced using the pJR1Ri vector, placing expression of the sense or antisense genes under the control of the CaMV 35S promoter and *nos* polyadenylation signal. These plasmids were transferred to *Agrobacterium tumefaciens* strain LBA4404 by tri-parental matings. Leaf disks from tobacco *Nicotiana tabacum* cv (Samsun NN) were inoculated with the transformed *Agrobacterium* strains and transgenic plants regenerated using standard protocols.

These plants were used in experiments using the bacterial elicitor, harpin (the *HrpN* gene product from *Erwinia amylovora*). In wild-type tobacco plants, and plants expressing an antisense GTase gene, harpin injection into leaves caused the formation of necrotic lesions, but such lesions were not observed in plants over-expressing the GTase (sense construct).
RNA was extracted from the injected leaves and the expression of PR genes assessed by Northern blotting (**FIG 15**). In wild-type plants, PR3a gene expression peaked at 1 day post-injection and was present at high levels throughout the time-course. In antisense plants, the peak at 1 day was maintained over the whole time course, whereas in over-expressing plants, PR3a expression was significantly suppressed.

These data suggest that a key signal which controls lesion formation and PR gene expression is affected by DCA GTase expression levels.

EXAMPLE 8

Phenolic compounds such as salicylic acid have been shown to be important signals during plant defence responses. To investigate the levels of phenolics in leaves of wild-type and transgenic tobacco plants, we subjected methanol extracts from leaves to HPLC analysis. Phenolic profiles analysed by ultra violet spectrophotometry and diode array detectors indicated increased levels of one specific compound in leaves of tobacco plants over-expressing the GTase in comparison to wild-type. This phenolic was identified on the basis of retention time and spectral characteristics as the glucoside of dehydrodiconiferyl alcohol, suggesting that the TWI1 gene may encode dehydrodiconiferyl alcohol glucosyl transferase (DCA GTase).

Recombinant protein was produced to test this. The tobacco DCA GTase cDNA clone was amplified by the PCR from pBluescript using oligonucleotide NTSG1 (5'-CGGGATCCATGGGTCAGCTCCAT-3') and T7 primer. The amplified fragment was digested by XhoI and filled in by Klenow fragment to create a 3' blunt end. Subsequently the DNA fragment was digested by BamHI and subcloned into the BamHI and SmaI sites of the prokaryotic fusion gene expression vector pGEX2T (Pharmacia) and the plasmid was named as pNTSG-2T. The glutathione-S-transferase (GST)/GTase fusion protein was expressed and purified according to the manufacturer's instructions. DCA was produced by peroxidation of commercially available coniferyl alcohol, and tested as a substrate for the GTase protein in an assay including UDP-glucose. Other potential substrates such as indole acetic acid (auxin) and salicylic acid were also tested. The recombinant protein was found to have glucosyl transferase enzyme activity only towards DCA, indicating that this is a specific DCA GTase. **FIG 16** shows typical HPLC traces obtained from the products of reactions conducted using protein purified from *E.coli.* transformed with the GST/GTase construct and with protein from *E. coli* transformed with an unrelated (annexin p35) GST fusion protein. Dehydrodiconiferyl alcohol glucoside is only present in the reaction products from the experiment including the recombinant GTase protein.

EXAMPLE 9

To express the tomato GTase, the cDNA encoding tomato GTase (TWI1) was released from the cDNA clone and subcloned into the EcoRI site of the expression vector pGEX3X (Pharmacia). The desired orientation construct was cleaved with BamHI and the linearized plasmid DNA was then blunt-ended with Klenow fragment and self-ligated, in order to obtain an in-frame fusion with the fusion domain. The final construct (pGEX-P8E) was transformed into *E. coli* and the fusion protein was expressed and tested as described in Example 8. **FIG 17** shows an HPLC trace indicating the glycosylation of DCA by the GST-TWI1 fusion protein.

EXAMPLE 10

The tomato TWI1 cDNA sequence can be used as a heterologous probe to identify the DCA GTase from a range of Solanaceous species as shown by Southern blotting. We have isolated full-length cDNA clones corresponding to the DCA GTase of tobacco using the tomato TWI1 sequence as a probe **(FIG 4)**. These two genes show around 85% identity in primary sequence. Additional GTases have been identified in DNA sequence databases of expressed sequence tags from rice **(FIG 5)**.


1. Doherty, HM, Selvendran, RR, Bowles, DJ (1988) *Physiol. Mol. Plant Pathol.* **33**; 377-384.
2. Doherty, HM, Bowles, DJ (1990) *Plant Cell Environ.* **13**, 851-855.
3. Pena-Cortes, H, Albrecht, T, Prat, S, Weiler, EW, Willmitzer, L (1993) *Planta* **191**; 123-128.
4. Doares, SH, Narvaez-Vasquez, J, Conconi, A, Ryan. CA (1995) *Plant Physiol.* **108**; 1741-1746.
5. Szerszen, JB, Szczyglowski, K, Bandurski, RS (1994) *Science* **265**; 1699-1701.
6. Yalpani, N, Schulz, M, Davis, MP, Balke, NE (1992) *Plant Physiol.* **100**; 457-463.
7. Ecker, JR, Davis, RW (1995) *Science* **268**:667-675.
8. Ward, ER, Uknes, SJ, Williams, SC, Dincher, SS, Wiederhold, DL, Alexander, DC, Ahl-Goy, P, Metraux, J-P, Ryals, JA (1991) *Plant Cell* **3**; 1085-1094.
9. Abeles, FB, Morgan, PW, Saltveit, ME Jr. "*Ethylene in plant biology*," Academic Press.
10. Wei, Z.M., Laby, R.J., Zumoff, C.H., Bauer, D.W., He, S.Y., Collmer,A., Beer, S.V. (1992) *Science* **257**: 85-88.
11. Gan, S, Amasino, RM (1995) *Science* **270**; 1986-1988.
12. Nicholass, FJ, Smith, CJS, Schuch, W, Bird CR, Grierson,D (1995) *Plant Mol. Biol.* **28**; 423-435.
13. Bevan, M (1984) *Nucleic Acids Res.* **12**; 8711.
14. Picton, S, Gray, J, Barton, S, Abu Bakar, U, Lowe, A, Grierson, D (1993) *Plant Mol. Biol.* **23**; 193-207.
15. O'Donnell, PJ, Calvert, CM., Atzorn, R., Wasterpack, C., Leyser, HMO., Bowles, DJ (1996) *Science* **274,** 1914-1917.


## Claims

1. The use of an isolated TWI1 gene in regulating the level of dehydroconiferyl alcohol glucoside (DCG) in plants, said TWI1 gene encoding dehydroconiferyl alcohol glucosyl transferase (DCA GTase).

2. The use of a TWI1 gene as claimed in claim 1, wherein said TWI1 gene comprises a nucleic acid sequence of FIG1 and/or FIG2 or comprises a nucleic acid sequence which hybridises under stringent conditions to the sequences of FIG1 and/or FIG2.

3. The use of a TWI1 gene as claimed in claim 1 or claim 2, wherein said TWI1 gene is isolated from tomato.

4. The use of a TWI1 gene as claimed in claim 1 or claim 2, wherein said TWI1 gene comprises the nucleic acid sequence of FIG4 and is isolated from tobacco.

5. The use of TWI1 gene as claimed in claim 1 or claim2, wherein said TWI1 gene comprises the nucleic acid sequences of FIG5 and is isolated from rice.

6. The use of TWI1 gene as claimed in any one of claims 1 to 5 in regulating levels of DCG, thereby altering the signalling pathways of salicylic acid, jasmonic acid and/or ethylene in plants.

7. The use of TWI1 gene as claimed in any one of claims 1 to 5 in regulating salicylic acid-mediated pathogen responses in plants.

8. The use of a TWI1 gene as claimed in any one of claims 1 to 6 in regulating levels of ethylene during senescence and fruit ripening.

9. The use of a TWI1 gene as claimed in any one of claims 1 to 8 in dicotyledonous and in monocotyledonous plants.

**10.** The use of TWI1 gene as claimed in any one of claims 1 to 9 in tomato plants.

**11.** A method of regulating the level of plant ethylene present during wounding or pest or pathogen infestation in plants, said method comprising regulating the level of plant DCA GTase by regulating TWI1 gene expression, said TWI1 gene encoded by the nucleic acid sequence comprised in FIG 1 or FIG2 or a nucleic acid sequence hybridising under stringent conditions to the sequence comprised in FIG1 or FIG2.

FIG 1

1 TCATTTTTTC TTCTTTCCCG ATGATGCTCA AGGTCATATG ATACCTACAC
51 TTGACATGGC GAACGTTGTC GCTTGTCGTG GTGTTAAAGC CACTATAATC
101 ACAACACCTC TCAATGAATC TGTTTTCTCT AAAGCTATTG AGAGAAACAA
151 GCATTTAGGT ATTGAAATTG ATATTCGTTT ACTAAAATTC CCAGCTAAGG
201 AGAATGATTT GCCTGAAGAT TGTGAGCGTC TTGATCTTGT ACCTTCTGAT
251 GACAAACTCC CAAACTTCTT AAAAGCTGCG GCTATGATGA AAGATGAATT
301 TGAGGAGCTT ATTGGAGAAT GTCGCCCTGA TTGTCTTGTT TCTGATATGT
351 TCCTTCCATG GACTACTGAT AGTGCAGCCA AATTTAGCAT ACCAAGAATT
401 GTATTCCATG GAACTAGTTA CTTTGCCCTT TGTGTTGGCG ATACGATCAG
451 GCGTAATAAG CCTTTCAAGA ATGTGTCATC GGATACTGAA ACTTTTGTTG
501 TACCGGATTT GCCACATGAA ATTAGGCTAA CTAGAACACA GTTGTCTCCG
551 TTTGAGCAAT CGGATGAAGA GACGGGTATG GCTCCCATGA TTAAAGCTGT
601 GAGGGAATCG GATGCGAAGA GCTATGGAGT TATATTCAAT AGCTTTTATG
651 AGCTTGAATC AGATTATGTT GAACATTACA CTAAGGTTGT AGGTAGAAAA
701 AATTGGGCTA TTGGTCCGCT TTCGCTGTGC AATAGGGATA TTGAAGATAA
751 AGCGGAAAGA GGGAGGAAAT CATCTATCGA TGAACACGCG TGCTTGAAAT
801 GGCTTGATTC GAAGAAATCA AGTTCCATTG TTTATGTTTG TTTTGGAAGT
851 ACAGCAGATT TCACTACAGC ACAGATGCAA GAACTTGCTA TGGGGCTAGA
901 AGCCTCTGGA CAAGATTTCA TTTGGGTTAT CAGAACAGGG AATGAAGATT
951 GGCTCCCAGA AGGATTCGAG GAAAGAACAA AAGAAAAAGG TTTAATCATA
1001 AGAGGATGGG CACCCCAAAG TGTGATTCTT GATCACGAAG CTATTGGAGC
1051 TTTTGTTACT CATTGTGGAT GGAACTCGAC ACTGGAAGGA ATATCAGCAG
1101 GGGTACCAAT GGTGACATGG CCAGTATTTG CGGAACAGTT TTTCAATGAG
1151 AAGTTGGTGA CTGAGGTAAT GAGAAGTGGA GCTGGTGTTG GTTCTAAGCA
1201 ATGGAAGAGA ACAGCTAGTG AAGGAGTGAA AAGAGAAGCA ATAGCAAAGG
1251 CGATAAAGAG AGTAATGGCG AGTGAAGAAA CAGAGGGATT CAGAAGCAGA
1301 GCAAAAGAGT ACAAAGAAAT GGCAAGAGAA GCTATTGAAG AAGGAGGATC
1351 ATCTTACAAT GGATGGGCTA CTTTGATACA AGACATAACT TCATATCGTT
1401 AACTAGTTGA TGCAAAAAAA GAAAAAACAT GTGTGTTTCT ATATTCTGTC
1451 TTCTGTTTTG CTGATTTGAT CATATTACGT ACTTCTTCAT GATAATTAAT
1501 GACATCAATA GAATCCAAGA TCAATCATCT CGAAATTCAA CGTTAAAATA
1551 TTTCGACATT TGAATAATAC ATCGACTTAA AATGGAAAAA AAAAAAAAAA
1601 AAAAAAAAAA AAAAAAAAAA AAAA

FIG 2

AAGCTTACAAGATAGTGTCATGTAGGCCGAAAAAGATAGAAAATTATTAATAAATTAAATTTAAGAGGT
AATATAACCTTATTATAATATAAATGTGTATCTAAAATTTCTGACATAAATCTAGGGAATAGTTATACA
TTATTCTTTATTATTATTATTGAGTCGTCAAAAAATATTATTAGAATTTATGAGCTAATACATATTTAA
TTTTATAATGTAAATATATTTTTTTAAAAATTTACCGACTTCAATAGAACCCCACGAACCTTATCTATA
TCCGCCTCGTGACCACCACCTTCTCAAGTATTCCGCCAAAATCAAATGGCAATTACCGGTTCCTACTGC
AATAATTTAGCAGCTAATGAACAAAATGCATCTTGTCATCTTCTAGATGATTTGTACTTCTTTCTGCTT
AATAATAATCGTTGACCGTTGATTTAACATAAAAAGACAAATGACTCGAAATAATGATTAAAAATAATA
AATGATAAAGGTTATCTTATTAAATAACTGTTACTAAACATAAGTCATTTAAATATTAAAAATCAATAT
TCTAATTTTTATGATAAATTTATTAAATTATTTATTAATTTTATAAATCAAATAAATATTATTGAATAT
CGGTATAATAGATGATGGACGGAGTGGATGTATCCAATGTCTCAATCGAACTGACTCGCACATGTATTT
TCGTCTTTGAACAAAACACTTGTCGTTAAATTAACTTAAATAATAAAAGGAAAGTTTGTGTTTCAATTG
ACTTGTGACGGAAGGGCATATGTCACCTGTAAAGTTACATCTCAAGAAATTCCATACGCTGGTCCCCGC
TATTGCCGGTTTCCTTCATTGACAAGGCCAGTTATTTTTGAGATTGAATTTATTTCACCTCCGATTATT
AGATATTTATTAAAATCGATATATGTTTAGTCATGATTTTATATATAAAATAGTTATGATCACGAAATA
TTTCGATCTTAAACTAATAAAACCTTGATGAGTTGTTTAGGGTTTGTCTGTTAAATCAACCACGTGAGT
GCTTACGTGACTTCTTTACGTCCAACTTGAAATAACAAGTTAATCTCTTATGCAACTTCAACGAAAGTC
TTCAAACAATTCGACGTATATATAACGTGACACTAATGCAATTACAACTCAAAAAGTATTTACTCCTCT
TTGTTTCATTCAAGCATTTCCACAA **ATG**

FIG 3

1 MGELHFFFFP DDAQGHMIPT LDMANVVACR GVKATIITTP LNESVFSKAI
51 ERNKHLGIEI DIRLLKFPAK ENDLPEDCER LDLVPSDDKL PNFLKAAAMM
101 KDEFEELIGE CRPDCLVSDM FLPWTTDSAA KFSIPRIVFH GTSYFALCVG
151 DTIRRNKPFK NVSSDTETFV VPDLPHEIRL TRTQLSPFEQ SDEETGMAPM
201 IKAVRESDAK SYGVIFNSFY ELESDYVEHY TKVVGRKNWA IGPLSLCNRD
251 IEDKAERGRK SSIDEHACLK WLDSKKSSSI VYVCFGSTAD FTTAQMQELA
301 MGLEASGQDF IWVIRTGNED WLPEGFEERT KEKGLIIRGW APQSVILDHE
351 AIGAFVTHCG WNSTLEGISA GVPMVTWPVF AEQFFNEKLV TEVMRSGAGV
401 GSKQWKRTAS EGVKREAIAK AIKRVMASEE TEGFRSRAKE YKEMAREAIE
451 EGGSSYNGWA TLIQDITSYR

# FIG 4

```
  1 AAGAACTGAA AACAACCACA CGTCTTTACT TTTCTTTCTG CTTTCTGATA
 51 CTAAACTACA TTTTTCTTTC TTTCATTCAA ACATTTTCAC AAATGGGTCA
101 GCTCCATTTT TTCTTCTTTC CTGTGATGGC TCATGGCCAC ATGATTCCTA
151 CGCTAGACAT GGCCAAGCTC GTTGCTTCAC GTGGAGTTAA GGCCACTATA
201 ATCACAACCC CACTCAATGA ATCCGTTTTC TCCAAATCTA TTCAAAGAAA
251 CAAGCATTTG GGTATCGAAA TCGAAATCCG TTTGATCAAA TTCCCAGCTG
301 TTGAAAATGG CTTACCTGAA GAATGCGAGC GCCTCGATCT CATCCCTTCA
351 GATGATAAGC TCCCAAACTT CTTCAAAGCT GTAGCTATGA TGCAAGAACC
401 ACTAGAACAG CTTATTGAAG AATGTCGACC CAATTGTCTT GTTTCTGATA
451 TGTTCCTTCC TTGGACTACT GATACTGCAG CCAAATTTAA CATGCCAAGA
501 ATAGTTTTTC ATGGCACAAG CTTGTTTGCT CTTTGTGTCG AGAATAGCAT
551 CAGGCTAAAT AAGCCTTTCA AGAATGTCTC CTCTGATTCT GAAACTTTTG
601 TTGTACCGAA TGTGCCTCAC GAAaTAAaTG ACCAGACCCA GTTG
```

FIG 5

```
1    CTTTCCCGGC CGCCGAGGCG CNTNANCCGG AGGGGTGCGA GAGGGTGGAC
51   CACGTCCCCT CGCCGGACAT GGTGCCGAGC TTCTTCGACG CCGCCATGCA
101  GTTCGGCGAC GCAGTGGCGC ANACTNCNGG CGCCTCACGG GGCCGCGCCG
151  GCTGAGCTGC CTCATCGCCG GGATATCTCA CACGTGGGCG CACGTCCTGG
201  CGCGCNACTC GGCGCTCCGT GCTTCATCTT CCACGGTTTC TGCGCGTTCT
251  CCCTGCTCTG CTGCNAGTAC CTGCACGCGC ACAGGCCGCA CGAGGCGGTC
301  TCCTCGCCGG ACGAGCTCTT TGACGTCCCT GTCCTGCCGN CTTTCGAGTT
351  CAGG
```

FIGURE 6

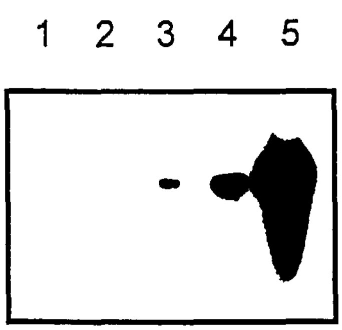

FIGURE 7

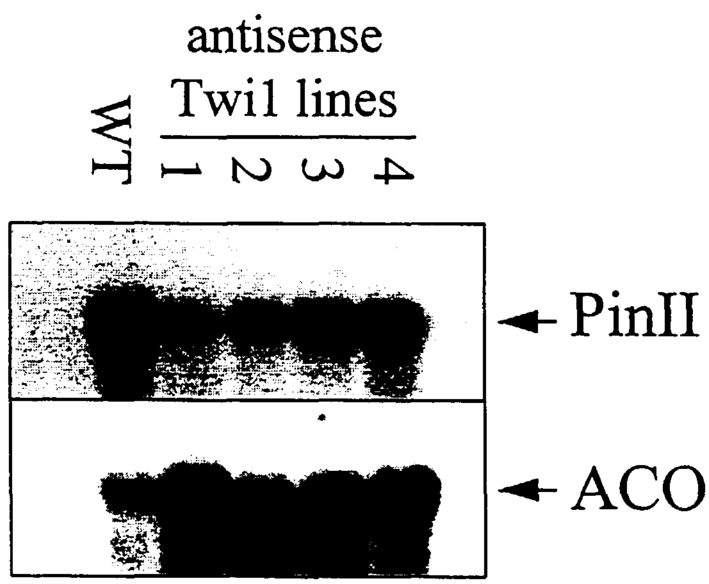

FIGURE 8

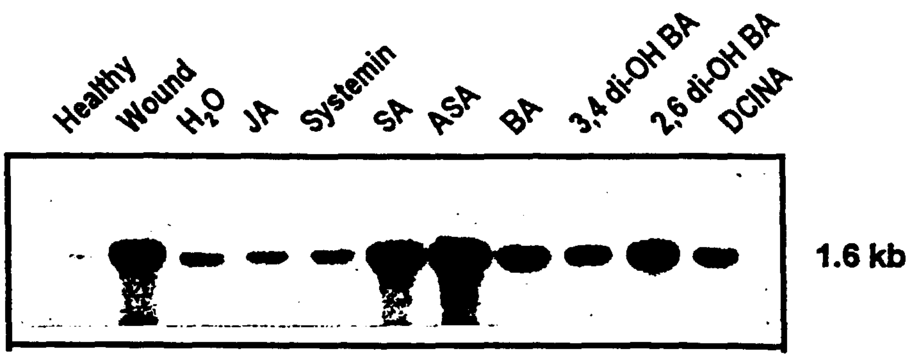
Healthy
Wound
$H_2O$
JA
Systemin
SA
ASA
BA
3,4 di-OH BA
2,6 di-OH BA
DCINA
1.6 kb

FIGURE 9

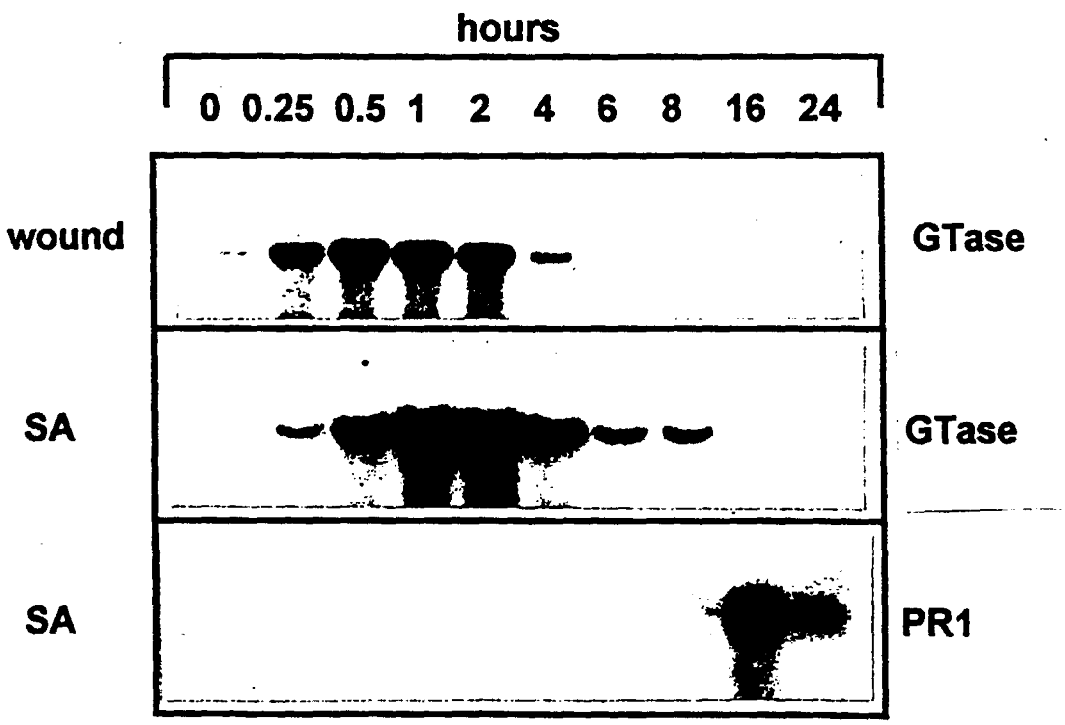

FIGURE 10

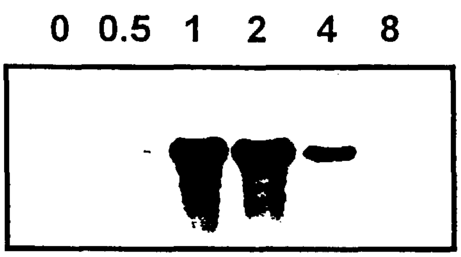

FIGURE 11

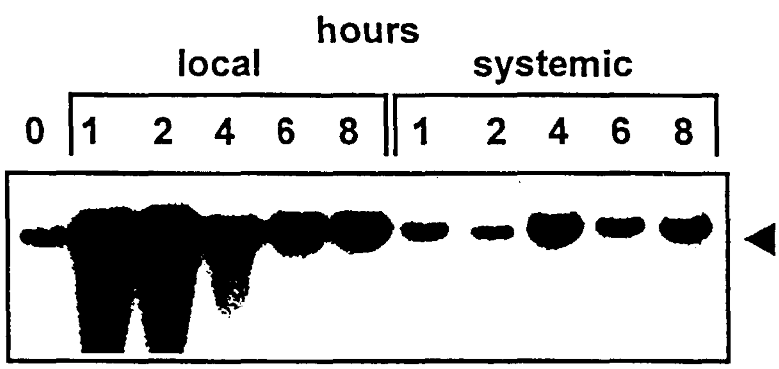

FIGURE 12

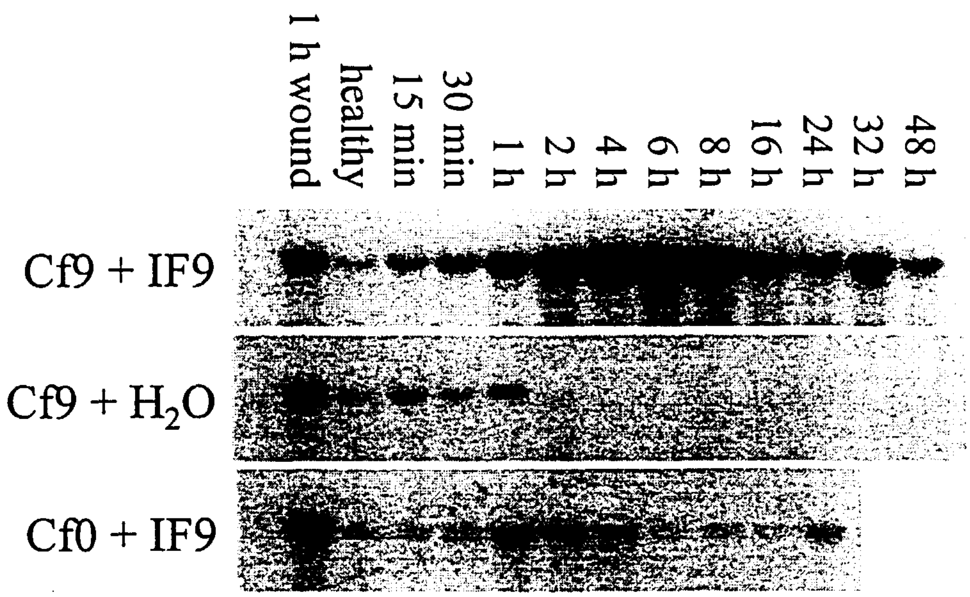
1 h wound
healthy
15 min
30 min
1 h
2 h
4 h
6 h
8 h
16 h
24 h
32 h
48 h
Cf9 + IF9
Cf9 + $H_2O$
Cf0 + IF9

FIGURE 13

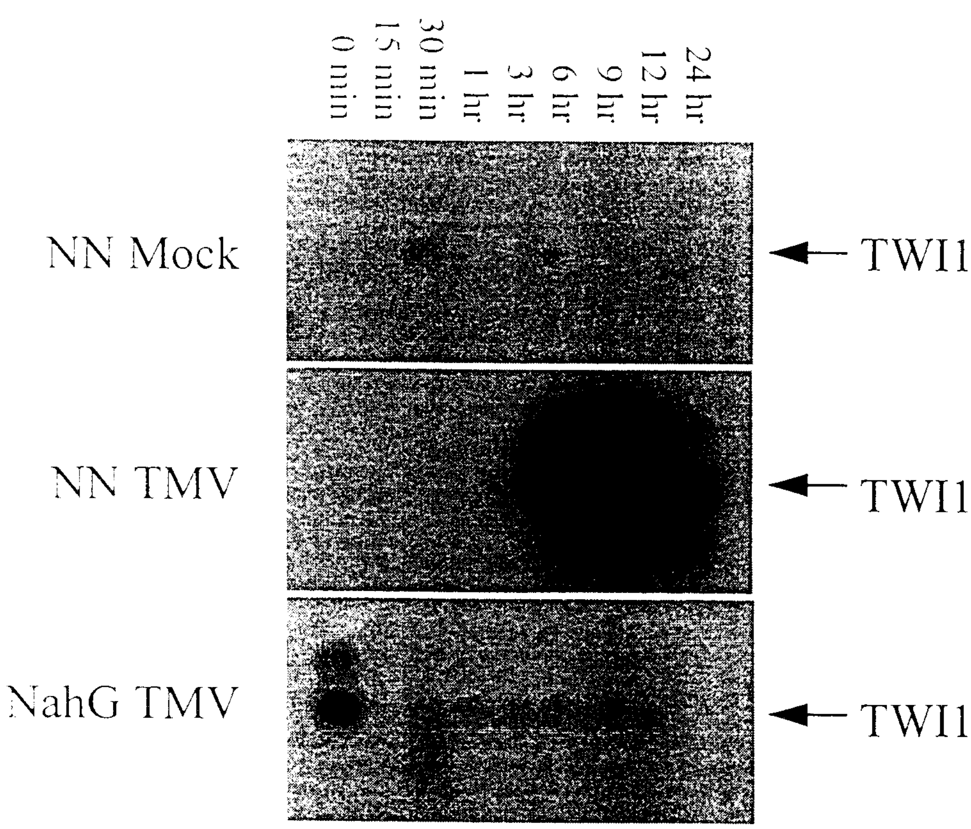
0 min
15 min
30 min
1 hr
3 hr
6 hr
9 hr
12 hr
24 hr
NN Mock
NN TMV
NahG TMV
TWI1
TWI1
TWI1

FIGURE 14

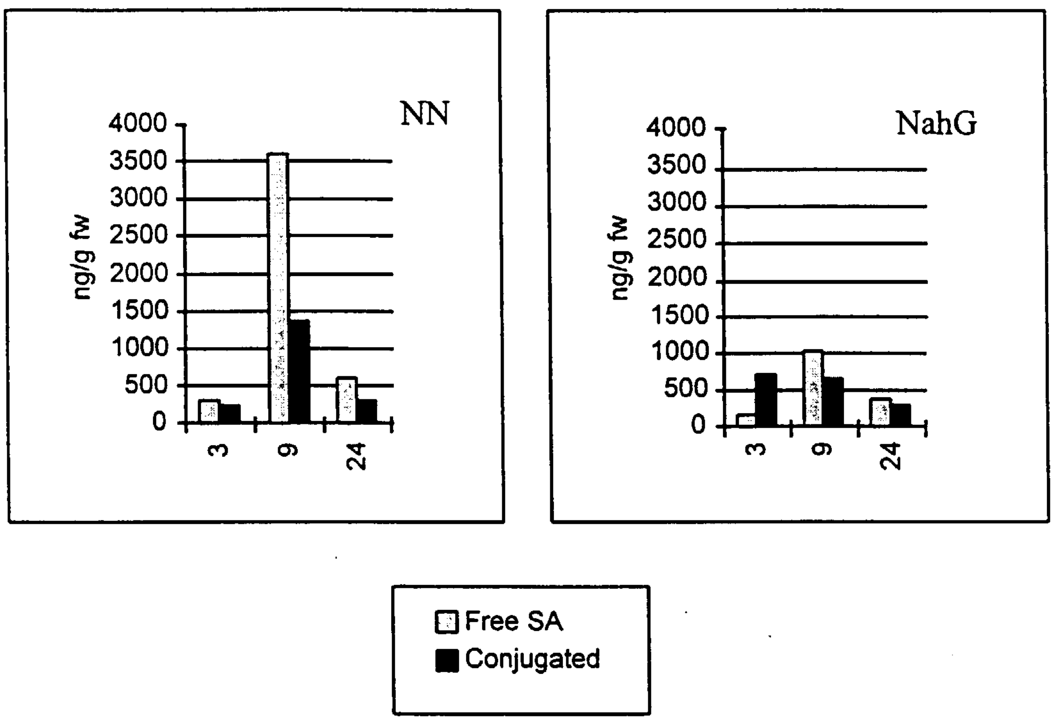
NN
4000
3500
3000
2500
2000
1500
1000
500
0
ng/g fw
3
9
24
NahG
4000
3500
3000
2500
2000
1500
1000
500
0
ng/g fw
3
9
24
Free SA
Conjugated

FIGURE 15

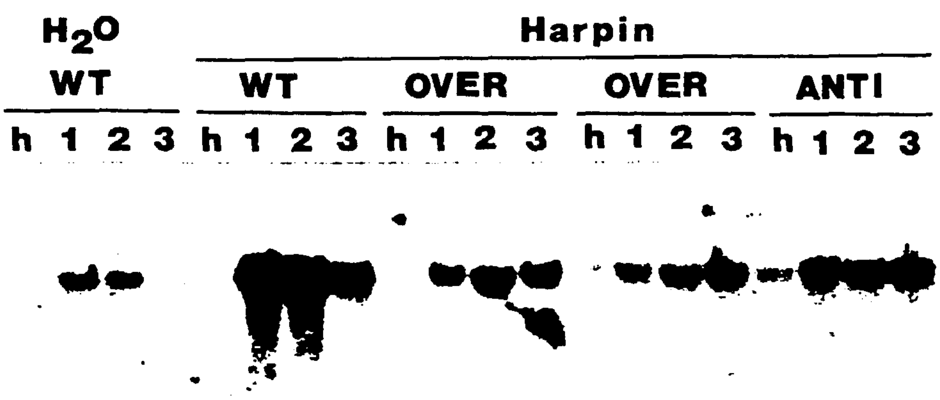

FIGURE 16

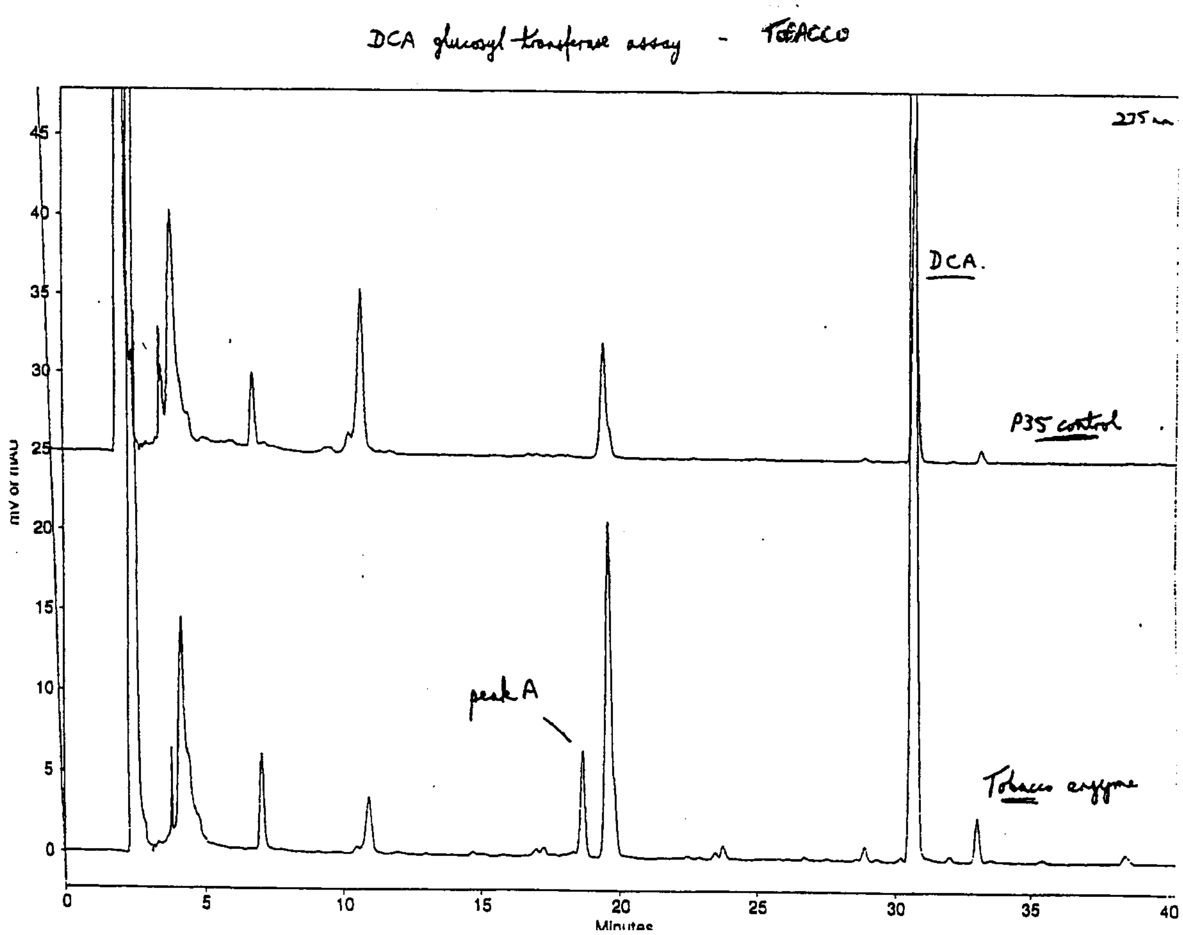
DCA glucosyl transferase assay - TOBACCO
275 nm
DCA.
P35 control
peak A
Tobacco enzyme
mV or mAU
45
40
35
30
25
20
15
10
5
0
0
5
10
15
20
25
30
35
40
Minutes

FIGURE 17

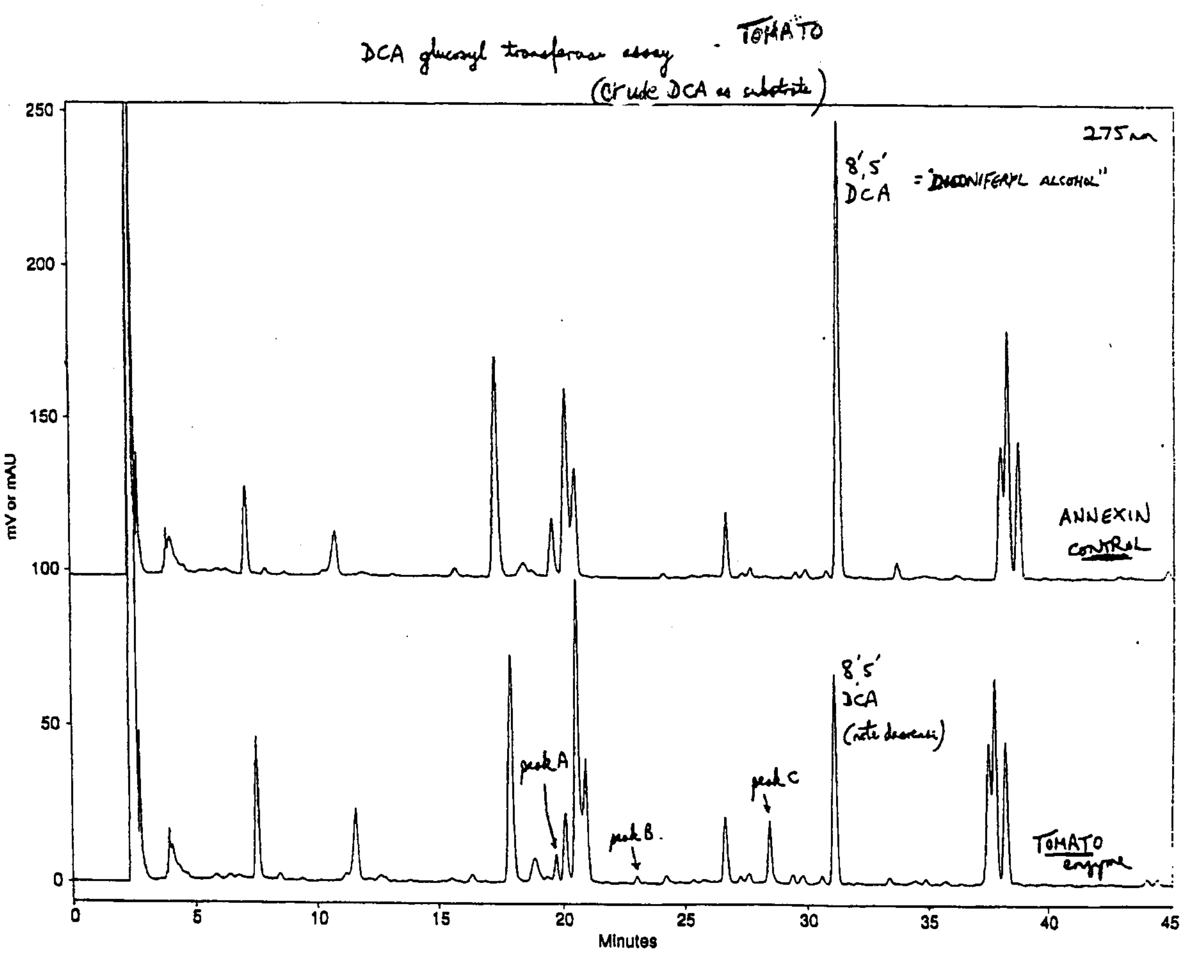
TOMATO
275nm
8',5'
DCA
ANNEXIN
CONTROL
8'5'
DCA
peak A
peak B
peak C
TOMATO
enzyme
250
200
150
100
50
0
mV or mAU
0
5
10
15
20
25
30
35
40
45
Minutes